# EUROPEAN PATENT APPLICATION

(11) **EP 1 498 132 A1**
(43) Date of publication of application: **19.01.2005**
(21) Application number: 03717664.1
(22) Date of filing: 21.04.2003
(51) Int. Cl.: A61K 35/78, A61P 11/02, A61P 11/06, A61P 17/00, A61P 37/08, A23L 1/30

(54) **COMPOSITIONS FOR PREVENTING OR TREATING POLLENOSIS, ALLERGIC NEPHRITIS, ATOPIC DERMATITITS, ASTHMA OR URTICARIA**

(30) Priority: 22.04.2002 US 126779
(71) Applicant: Matsuura Yakugyo Co., Ltd., Nagoya-shi, Aichi 460-0002 (JP)
(72) Inventor: YOSHIDA, Satoshi, Itabashi, Tokyo 175-0082 (JP)
(74) Representative: HOFFMANN - EITLE
(86) International application number: PCT/JP2003/005050
(87) International publication number: WO 2003/088988

(57) **Abstract**

A method for prevention or curative of pollen allergy, allergic rhinitis, atopic dermatitis, asthma or urticaria by administration of two kinds of crude drugs - *Cucurbita moschata* seeds and flowers of *Carthamus tinctorius -* and at least one crude drug selected from *Plantago asiatica, Lonicera japonica, Glycyrrhiza uralensis, Coix lachrymal-jobi* var. *ma-yuen, Zingiber officinale, Curcuma longa, Curcuma zedoaria* and *Artemisia argyi* to a patient; and a health food for prevention, or improvement, or reduction of these symptoms containing the above substances.

## Description

### Technical Field

The present invention is a preventive or curing composition for pollen allergy, allergic rhinitis, atopic dermatitis, asthma or urticaria, a health food for prevention, or a functional food, which contains as an effective component two crude drugs of pumpkin seeds and safflower therein.

### Background Art

In recent years, there has been a rapid increase in the number of patients suffering from allergies caused by pollen from plants such as cedar pollen, *Ambrosia artermisiifolia, Dactylis glomerata, Phleum pratense.* The symptoms of pollen allergies are observed particularly in the nose and eyes, and appear as allergic conjunctivitis and allergic rhinitis (such as sniffles, stuffiness and sneezing) due to chemical transmitters such as histamine and leukotriene and various enzymes that are liberated from mast cells and basophils when pollen invades into the body.

There has been also an increase in allergic rhinitis (THE MERCK MANUAL 16th ed. , PP. 326 to 327), atopic dermatitis (THE MERCK MANUAL 16th ed., PP. 2409 to 2410), asthma (THE MERCK MANUAL 16th ed. , P. 652), urticaria (THE MERCK MANUAL 16th ed. , PP. 322 to 323), and the like caused not only by pollen, but also by house dust, ticks, and the like as an allergen.

As a curing method therefore, there have been adopted preventive treatment by anti-allergic agents, or symptomatic treatments by new generation antihistaminic agents having a fewer sedation action, orally administered agents such as various antagonists to chemical transmitters and inhibitors of the liberation of chemical transmitters, steroidal agents having a rapid pharmaceutical effect, and antihistaminic nasal drops having immediate effect and strong action or desensitization therapy, or the like. However, Chinese drugs or antiallergic agents having a satisfactory effect in preventive treatment have not been developed yet. In addition, with regard to antihistaminic agents and steroidal preparations, which are used for symptomatic treatments, their side effects often cause problems. In terms of therapy, it has been also reported that the degree of patients' satisfaction with the current status of treatment is extremely low. On the other hand, the desensitization therapy, while having been appreciated again to a certain extent, is beginning to show its limitations.

It is disclosed that, as far as *Cucurbita moschata* seeds, *Plantago asiatica,* and *Lonicera japonica* are concerned, when one or more (particularly three) of *Cucurbita moschata* seeds, *Plantago asiatica,* and *Lonicera japonica* are added to feed, natural infections caused by parasites, bacteria and viral diseases are largely prevented, the prophylactic force of living bodies is enhanced, and the flesh and an egg quality are improved. Furthermore, it is disclosed that a feed into which crude drugs of *Cucurbita moschata* seeds, *Plantago asiatica, Lonicera japonica,* and Carthamus tinctorius are incorporated can show the improvement in the health conditions, survival rates, an improvement of an egg quality, and the effects of anti-leucocytozoonosis in layers; and also the effects of anti-Newcastle disease, and the inhibitory effects of the number of enteric Coccidium and *Staphylococci* in the intestine in quail (See Patent Document 1, for Example).

There is disclosed a method for producing an interferon inducer from the plants of the genus *Cucurbitaceae,* such as Japanese pumpkin (See Patent Document 2, for example). There is disclosed an antiviral activity and anti-tumor activity of interferon inducers extracted from *Carthamus tinctorius* (See Patent Document 3, for example). In addition, it is disclosed that interferon inducers may be extracted from the flowers of *Lonicera japonica,* seeds of *Plantago asiatica,* etc. , and are useful for the preventive and curing treatments of viral infections in human beings and animals (See Patent Document 4, for example). There is also disclosed a macrophage activator having been incorporated with two crude drugs of *Cucurbita* *moschata* seeds and *Carthamus tinctorius* (See Patent Document 5, for example). There is disclosed a neutrophil activator wherein four kinds of crude drugs *Cucurbita moschata* seeds, *Carthamus tinctorius, Plantago asiatica, Carthamus tinctorius,* and *Lonicera japonica* have been incorporated (See Patent Document 6, for example).

However, there are neither teachings nor implications as to the preventive or curative treatments for diseases in human beings by the combination of the crude drugs that are the effective components of the present invention in any of those documents, while there is disclosed the interferon-inducing effects, macrophage-activating effects, neutrophil activating effects, or inhibitory action of IgE anti-body production as to those crude drugs:
[Patent Document 1]
   US Patent No. 5,882,672 specification
[Patent Document 2]
   US Patent No. 4,421,746 specification
[patent Document 3]
   US Patent No. 4,456,597 specification
[Patent Document 4]
   US Patent No. 4,469,685 specification
[Patent Document 5]
   JP-A-11-116498 Laid-open specification, and
[Patent Document 6]
   JP-A-11-116498 Laid-open specification.

### Disclosure of the Invention

The present inventors have carried out further an intensive investigation to find the concrete usage of crude drugs of *Cucurbita moschata* seeds, *Plan tago asiatica, Carthamus tinctorius, Lonicera japonica,* and the like. As a consequence, it has been confirmed that the crude drug combination of the present invention, at least the combination of *Cucurbita moschata* seeds, and *Plantago asiatica* can improve to a great extent the symptoms of the patients who are affected with pollen allergy, allergic rhinitis, atopic dermatitis, asthma or urticaria, or can prevent the development thereof to a great extent. Furthermore, it has been confirmed that the combined crude drugs of the present invention is quite safe, and that it does not cause any side effect even if the combined crude drugs have been continuously and habitually taken for a long period of 6 years, and has been found that the combined crude drugs are suitable for a continuos and habitual use of a long period of time not only as a curing medicine, but also as a preventive medicine, a health food, or a functional food.

Thus, the combination of the present inventive crude drugs is a combined composition of two kinds of crude drugs of *Cucurbita moschata* seeds and *Carthamus tinctorius* and at least one crude drug selected from *Plantago asiatica, Lonicera* *japonica, Glycyrrhiza uralensis, Coix lachrymal-jobi* var. *ma-yuen, Zingiber officinale, Curcuma longa, Curcuma zedoaria* and *Artemisia argyi .* That is, the present invention can be described as follows:
1. A preventive or curing composition for pollen allergy, allergic rhinitis, atopic dermatitis, asthma or urticaria; containing two kinds of crude drugs of *Cucurbita moschata* seeds and *Carthamus tinctorius,* and at least one crude drug selected from *Plantago asiatica, Lonicera japonica, Glycyrrhiza uralensis, Coix lachrymal-jobi* var. *ma-yuen, Zingiber officinale, Curcuma longa, Curcuma zedoaria* and *Artemisia argyi.*
2. A composition as described in the aforementioned item 1 which is used for preventive or curative treatment of allergic rhinitis, asthma or urticaria.
3. A preventive or curative composition as described in the aforementioned item 1 or 2 which contains four kinds of crude drugs of *Cucurbita moschata* seeds, *Carthamus tinctorius, Plantago asiatica,* and *Lonicera japonica* as an effective component.
4. A preventive or curative composition for pollen allergy which is used for being administered to a mammal who suffers from pollen allergy yearly before the season of pollen allergy; containing two kinds of crude drugs of *Cucurbi ta moschata* seeds, *Carthamus tinctorius,* and at least one crude drug selected from *Plantago asiatica, Lonicera japonica, Glycyrrhiza uralensis, Coix lachrymal-jobi* var. *ma-yuen, Zingiber officinale, Curcuma longa, Curcuma zedoaria* and *Artemisia argyi* as an effective component.
5. A preventive composition as described in the aforementioned item 4 which contains four crude drugs of *Cucurbita moschata* seeds, *Carthamus tinctorius, Plantago asiatica, Lonicera japonica* as an effective component.
6. A preventive composition as described in any one of the aforementioned item 4 or 5 wherein the mammal is human being.
7. A method for preparing a preventive or curative composition for pollen allergy, allergic rhinitis, atopic dermatitis, asthma or urticaria; containing two kinds of crude drugs of *Cucurbita moschata* seeds and *Carthamus tinctorius,* and at least one crude drug selected from *Plan tago asiatica, Lonicera japonica, Glycyrrhiza uralensis, Coix lachrymal-jobi* var. *ma-yuen, Zingiber officinale, Curcuma longa, Curcuma zedoaria* and *Artemisia argyi.*
8. A method for preparing a preventive or curative composition for pollen allergy which is used for administering two kinds of crude drugs of *Cucurbita moschata* seeds, *Carthamus tinctorius,* and at least one crude drug selected from *Plantago asiatica, Lonicera japonica, Glycyrrhiza uralensis, Coix lachrymal-jobi* var. *ma-yuen, Zingiber officinale, Curcuma longa, Curcuma zedoaria* and *Artemisia argyi* to a patient who suffers from pollen allergy every year before the beginning of pollen allergy.
9. A health food or a functional food for the prevention of or the curing for pollen allergy, allergic rhinitis, atopic dermatitis, asthma or urticaria by administering two kinds of crude drugs of *Cucurbita moschata* seeds and *Carthamus tinctorius,* and at least one crude drug selected from *Plantago asiatica, Lonicera japonica, Glycyrrhiza uralensis, Coix lachrymal-jobi* var. *ma-yuen, Zingiber officinale, Curcuma longa, Curcuma zedoaria* and *Artemisia argyi* and *Lonicera japonica* as an effective component.
10. A health food or a functional food as described in the aforementioned item 9 which contains four kinds of crude drugs of *Cucurbita moschata* seeds, *Carthamus tinctorius, Plantago asiatica,* and *Lonicera japonica.*
11. A composition as described in any one of the aforementioned item 9 to 10 wherein the composition is used for preventing from, or improving or lightening pollen allergy, allergic rhinitis, atopic dermatitis, asthma or urticaria.
12. A health food or a functional food for the prevention of pollen allergy which contains two kinds of crude drugs of *Cucurbi ta moschata* seeds and *Carthamus tinctorius,* and at least one crude drug selected from *Plantago asiatica, Lonicera japonica, Glycyrrhi za uralensis, Coix lachrymal-jobi* var. *ma-yuen, Zingiber officinale, Curcuma longa, Curcuma zedoaria* and *Artemisia argyi* and *Lonicera japonica* as an effective component.
13. A health food or a functional food as described in the aforementioned item 12 which contains four kinds of crude drugs of *Cucurbita moschata* seeds, *Carthamus tinctorius, Plantago asiatica,* and *Lonicera japonica.*
14. An animal health food or an animal functional food for the prevention of pollen allergy allergic rhinitis, atopic dermatitis, asthma or urticaria, which contains two kinds of crude drugs of *Cucurbita moscha ta* seeds and *Carthamus tinctorius,* and at least one crude drug selected from *Plantago asiatica, Lonicera japonica, Glycyrrhiza uralensis, Coix lachrymal-jobi* var. *ma-yuen, Zingiber officinale, Curcuma longa, Curcuma zedoaria* and *Artemisia argyi* and *Lonicera japonica* as an effective component.
15. A composition, a health food, or a functional food as described in any one of the aforementioned items 1 to 14, wherein two crude drugs of 20 to 60 % by weight of *Cucurbita moschata* seeds and 10 to 40 % by weight of *Carthamus tinctorius,* and a 5 to 50 % by weight of at least one crude drug selected from *Plantago asiatica, Lonicera japonica, Glycyrrhiza uralensis, Coix lachrymal-jobi* var. *ma-yuen, Zingiber officinale, Curcuma longa, Curcuma zedoaria* and *Artemisia argyi* is a compounding ratio therein.
16. A composition, a health food, or a functional food as described in the aforementioned item 15, wherein 30 to 50 % by weight of the *Cucurbita moschata* seeds, 10 to 30 % by weight of *Carthamus tinctorius,* 5 to 30 % by weight of *Plantago asiatica,* and 5 to 30 % by weight of *Lonicera japonica* is a compounding ratio therein.
17. A method for preventing a patient from pollen allergy, allergic rhinitis, atopic dermatitis, asthma or urticaria, or curing him therefrom by administering an effective amount of two crude drugs of *Cucurbita moschata* seeds and *Carthamus tinctorius,* and at least one crude drug selected from *Plantago asiatica, Lonicera japonica, Glycyrrhiza uralensis, Coix lachrymal*-*jobi* var. *ma-yuen, Zingiber officinale, Curcuma longa, Curcuma zedoaria* and *Artemisia argyi.*
18. A method for preventing a patient from pollen allergy, allergic rhinitis, atopic dermatitis, asthma or urticaria or curing him therefrom by administering an effective amount of crude drugs of *Cucurbita moschata* seeds, *Carthamus tinctorius, Plantago asiatica,* and *Lonicera japonica.*
19. A method for preventing or curing a patient as described in the aforementioned item 17 or 18, wherein the method is to prevent a person from allergic rhinitis, atopic dermatitis, asthma or urticaria, or to cure him therefrom.
20. A use for preparing a preventive agent or curative agent of pollen allergy, allergic rhinitis, atopic dermatitis, asthma or urticaria, as an effective component, of two crude drugs of *Cucurbi ta moschata* seeds and *Carthamus, tinctorius,* and at least one crude drug selected from *Plantago asiatica, Lonicera japonica, Glycyrrhiza uralensis, Coix lachrymal-jobi* var. *ma-yuen, Zingiber officinale, Curcuma longa, Curcuma zedoaria* and *Artemisia argyi .*
21. A use for preparing a composition according to the aforementioned item 20 for preventive agent or curative agent of allergic rhinitis, atopic dermatitis, asthma or urticaria.
22. A use for preparing a preventive agent or curative agent as described in any one of the aforementioned item 20 or 21, which contains as an effective component *Cucurbita moschata* seeds, *Carthamus tinctorius, Plantago asiatica,* and *Lonicera japonica .*

In addition, its concomitant use with conventional curative agents not only for pollen allergy, allergic rhinitis, atopic dermatitis, asthma or urticaria, but also the curative agents for the diseases other than the above-mentioned diseases is presumed not to give any bad influence on the therapy. Therefore, its concomitant use with currently used curative agents in an auxiliary manner is thought to be possible.

The present invention will now be discussed in more detail as hereunder.

First, the crude drugs used in the present invention will be described.

*Cucurbita moschata* seeds (Nankashi) is a seed of the plant belonging to the genus *Cucurbitaceae,* however, seeds of similar plants capable of achieving the object of the present invention are included herein as well, in the present invention. Although *Cucurbita moschata* seeds may be used in their raw state, dry seeds are preferred because of their superior keeping qualities as medicament and health food. It is also possible to use the seed coat only. Cucurbitin, protein and vitamins A, B₁, B₂ and C, and carotene, and the like are contained as components therein.

*Carthamus tinctorius* (safflower) is a dried tubular flower of a plant belonging to the genus *Compositae.* It contains as a component Carthamin, safflor yellow, lignan and sterol. It is used for the curing of circulation disorders such as female reproductive disorders, sensitivity to the cold, menopausal disorders, etc.

*Plantago asiatica* is a member of the *Plantaginacea* and its matured seeds *(shazenshi)* or entire plant *(shazen)* are used. They contain polysaccharides, plantenolic acid, succinic acid, adenine, aucubin, plantaginin, vitamins A and B₁, etc. They are used for anti-inflammatory agents, diuretic agents and antidiarrheal agents as a crude drug.

*Lonicera japonica* (Suikazura) is of the genus *Gramineae* and its flower or bud, leaf, stem or entire plant is used. It contains inositol, tannin, saponin, lonicerin, etc., as components. It is used for an antipyretic agent, poison antidote, diuretic agent, anti-inflammatory agent, etc., as a crude drug.

*Glycyrrhiza uralensis* is one of the *Leguminosae* and its dried root and rhizome are used. Its main medicinal component is glycyrrhizin. It is used for an alleviant, remitting agent, antitussive agent, analgesic agent and expectorant as a crude drug.

*Coix lachrymal-jobi* var. *ma-yven* is of the *Gramineae* and its cereal is used. It contains fatty oil as one of its components and in the oil coixenolide, coixol, amino acids, vitamin B₁ and saccharides are contained. It is used for a cancer cell suppressor, antipyretic agent, sedative, tranquilizer and hypoglycemic agent as a crude drug.

*Zingiber officinale* is one of the *Zingiberaceae* and its dried rhizome is used. As raw ginger, the same effect is possible, although dried ginger is preferred as a medicament and health food in view of its preservability. It contains essential oils and gingerol. It is used for improvement of blood circulation, hypertension, the excitation of vasomotor centers, and excitation of sympathetic nerves as a crude drug.

*Curcuma longa* is of the genus *Zingiberaceae,* and its rhizome is used. It contains curcumin, and essential oils etc. , among its components. It is used for a cholagogue, hypotensive agent, antibacterial agent, sedative, etc., as a crude drug.

*Curcuma zedoaria* is of the *Zingiberaceae* and its rhizome is used. It contains curcumin, essential oils, etc., as the main components. It is used for an anti-tumor agent, antibacterial agent and stomachic agent as a crude drug.

*Artemisia argyi* is a member of the *Compositae* and its dried leaves are used. It contains essential oils and vitamins A, B, C and D as the main components. It is used for an antibacterial agent, agent for stopping bleeding and shortening blood coagulation time, antitussive agent, expectorant, anti-inflammatory agent, appetite promoter, etc., as a crude drug.

The patient (s) means in the present invention a mammal (s) suffering from a disease, preferably inclusive of human being, or ape, companion animals such as dog, cat or the like.

In the present invention, when these crude drugs are used as in the form of crude powder, fresh ones, dried ones in shadow, or dried ones are used, and they are cut into a piece or powdered. One may use an extract of these crude powders with water or organic solvents. That is, they are used in the form of crude powder, solvent preparation, powdery preparation, molded preparation, exudate, or the like. Alcohol, acetone, and the like or a mixture of two or more of them or a mixture with water of any one of them may be used therefor. In the extraction, several times as much solvent is added to the crude drug, and the extraction or percolation is carried out at a normal temperature or with heating. One may compound an extracted essence of each crude drug which has been extracted individually, or prepare an essence by subjecting a mixture of plural number of crude drugs mixed in advance to extraction.

The above-mentioned powder of crude drug or extract with water or with organic solvent may be utilized as health food, functional food (dietary supplement) or medicament as it is, or after making into various forms by a method well known.

For example, medicament or functional food (dietary supplement) may be provided in the form of tablets, diluted powder, fine granules, capsules, pills or syrup for oral use by means of a conventional method of preparation. For making into preparations, it is possible to add fillers, binders, disintegrating agents, lubricants, buffers, corrigents, stabilizers, etc., thereto. At least one inert diluent such as lactose, mannitol, glucose, hydroxypropyl cellulose, finely crystallized cellulose, starch, polyvinylpyrrolidone or magnesium metasilicate aluminate is mixed therewith. In addition to the inert diluents, the composition may contain additives such as lubricants such as magnesium stearate, starch or talc, disintegrating agents such as calcium cellulose glycolate, stabilizers such as lactose and solubilizing aids such as glutamic acid or aspartic acid in accordance with conventional methods. Tablets or pills may be coated, if necessary, with a sugar coat or with the film of a substance which is soluble in the stomach or in the intestine such as sucrose, or gelatin, agar, pectin, hydroxypropyl cellulose, hydroxypropyl methyl cellulose, and the like.

To such an extent that the crude drug which is the effective component of the present invention is not affected, it is also possible to further compound caffeine, water-soluble vitamins such as vitamin B₁, vitamin B₂, vitamin B₆, vitamin B₁₂, vitamin C, biotin, carnitine, pantothenic acid, nicotinic acid and derivatives thereof, fat-soluble vitamins such as vitamin A, vitamin E and derivatives thereof, and amino acids such as taurine and arginine therewith. It is further possible that oriental herbs (such as licorice root, ginkgo, dandelion, chrysanthemum flower, carrot, cinnamon, etc.) or western herbs (such as *Serenoa repens, Hypericum perforatum,* echinacea, *aniseed,* annual chamomile (chamomile), rosemary, mint, eucalyptus, lavender, rose, hibiscus, aloe, etc.) may be added in a supplementary manner. It is also preferred to combine the crude drug with *Perilla frutescens* var. *crispa,* which has been reported to be particularly effective for pollen allergy, allergic rhinitis and atopic dermatitis *(FOOD Style,* 21, 2(4), 50-54, 1998) in an supplementary manner.

Liquid compositions for oral use contain a pharmaceutically acceptable emulsion, solution, suspension, syrup, elixir, etc., and contain a commonly used inert diluent such as pure water or ethanol. In addition to the inert diluent, the composition may also contain a moisturizer, an auxiliary agent such as a suspending agent, sweeteners, flavor agents, aromatic agents and antiseptic agents.

In the case of health food, it is possible to provide the compound in the form of a beverage or confection such as a jelly, biscuit, cookie, candy, etc.

The present invention consists of *Cucurbita moschata* seeds, flowers of *Carthamus tinctorius,* and at least one kind of crude drugs from *Plantago asiatica, Lonicera japonica, Glycyrrhiza uralensis, Coix lachrymal-jobi* var. *ma-yuen, Zingiber officinale, Curcuma longa, Curcuma zedoaria* and *Artemisia argyi ,* as an effective component. It is preferable that the present inventive composition contains *Cucurbita moschata* seeds in a range of from 20% to 60% by weight, *Carthamus tinctorius* in a range of from 10% to 40% by weight, and each of the other crude drugs listed above in a range of from 5% to 50% by weight. More preferably, the present inventive composition contains *Cucurbita moschata* seeds in a range of from 20% to 60% by weight, *Carthamus tinctorius* in a range of from 10% to 40% by weight, and each of the other crude drugs listed above in a range of from 5% to 50% by weight. Further preferably, the present inventive composition contains *Cucurbita moschata* seeds in a range of from 20% to 60% by weight, *Carthamus tinctorius* in a range of from 10% to 40% by weight, *Plantago asiatica* in a range of from 5% to 50% by weight, and *Lonicera japonica* in a percentage of from 5% to 50% by weight (The total amount of *Cucurbi ta moschata* seeds, *Carthamus tinctorius, Plantago asiatica.* and *Lonicera japonica* will be made to be 100 % by weight as a whole).

The most preferable composition therefor is 30% to 50 % by weight of *Cucurbi ta moschata* seeds, 10% to 30% by weight of *Carthamus tinctorius,* 5% to 30% by weight of *Plantago asiatica* in a percentage of from 5% to 50% by weight, and 5% to 30% by weight of *Lonicera japonica.*

The combination of crude drugs according to the present invention may be appropriately determined for each case, taking age, sexuality, etc., of the subject into consideration. Usually, when 0.5-2 g or, preferably a total amount of 1 g of the crude drugs a day to a 60kg adult is orally administered; the desired curative or preventive effect can be achieved without any side effects.

The period for the administration of the present inventive crude drug prior to the onset of the pollen allergy to mammals who suffers from said allergy every year varies, depending upon the state of the patients, the time when the pollens of plants such as cedar, Japanese cypress, graminaceous plants such as orchard grass and timothy, Asteraceous plants such as common ragweed and mugwort, birch(Betula sp. ), and the like are scattering, and the like. For instance, in the case of pollen of cedar, it is said that the allergic symptoms appear from February to April. Thus, the administration period therefor is the period from December to Early February, preferably January to Early February.

The combination of crude drugs according to the present invention can be used not only as a preventive/curative agent and health food for humans, but also as a curative agent and health food for the improvement/reduction of health problems in companion animals such as dogs and cats.

### Brief Description of the Drawings

Fig. 1 shows the total amount of IgE antibodies in the blood produced in response to cedar pollen in a mouse to which the hot water extract of Formulation A was orally administered or intraperitoneally injected.
Fig. 2 shows the evaluation of the degree of improvement in patients suffering from pollen allergy effected by the hot water extracts of Formulation A in Preparation Example 1.
Fig. 3 shows the evaluation of the degree of improvement in patients suffering from pollen allergy effected by the hot water extracts of Formulation B in Preparation Example 1.
Fig. 4 shows the degrees of the improvement by the hot water extract of Formulation A in Preparation Example 1 in the symptoms that were observed every year but did not appear during the test period.
Fig. 5 shows the degrees of the improvement by the hot water extract of Formulation B in Preparation Example 1 in the symptoms that were observed every year but did not appear during the test period.
Fig. 6 shows the degrees of the improvement in the symptoms of pollen allergy by the hot water extract of Formulation B in Preparation Example 1 in that case its administration started prior to the appearance of the symptoms.
Fig. 7 shows the degrees of the improvement in the symptoms of pollen allergy by the hot water extract of Formulation B in Preparation Example 1 in that case its administration started after the appearance of the symptoms.
Fig. 8 shows the degrees of the improvement in the symptoms of pollen allergy by the hot water extract of Formulation B in Preparation Example 1 in male patients.
Fig. 9 shows the degrees of the improvement in the symptoms of pollen allergy by the hot water extract of Formulation B in Preparation Example 1 in female patients.

### Best Mode for Carrying Out the Invention

As hereunder, the present invention will be illustrated in more detail by way of Preparation Examples and Test Examples, although the present invention is not limited to those Examples, etc.

### Preparation Example 1.

Powder of crude drugs having the combined components of the following Formulation A or Formulation B was mixed and extracted using a 10-fold amount of water at 95 ± 5°C for 30 minutes, the extract was strained and condensed, then excipients such as reducing maltose, lactose, starch, etc. , and scent were added thereto and the mixture was subjected to a granulating step to produce fine granules:
Formulation A: components ratio: *Cucurbita moschata* seeds (50%), *Carthamus tinctorius* (20%), *Plantago asiatica* (15%), and *Lonicera japonica* (15%), and
Formulation B: components ratio: *Cucurbita moschata* seeds (40%), *Carthamus tinctorius* (20%), *Coix lachrymal-jobi* var. *ma-yuen* (20%) and *Perilla frutescens* var. *crispa* (20%).

### Preparation Example 2.

5.0 g of *Cucurbita moschata* seeds, 3.0 g of *Carthamus tinctorius,* 1.0 g of *Plantago asiatica,* 3.0 g of *Lonicera japonica,* 67 g of lactose, and 16 g of starch were mixed thoroughly in a vertical type blender, the resultant was mixed with a kneading solvent that had been prepared in advance by dissolving 2 g of hydroxypropyl cellulose and 5 g of capric acid triglyceride into 40 g of 85 % aqueous ethanol solution, thus kneaded mixture was granulated in a basket type granulator ( screen diameter of 1 mm) . Thereafter, the resultant was passed through a sieve of 14 mesh, and passed ones were dried to give columnar like granules. Then, the aforementioned components were thoroughly mixed with mannite, hydroxypropyl cellulose, magnesium metasilicate-aluminate, aspartame, and scent to give 12 packages of fine granules (See JP-A-200-231584).

One may prepare compositions containing the components in various ratios in the same manner as that of Preparation Example 2, as shown below:

**Table 1**

| Preparation Example | *Cucurbita moschata* seeds | *Carthamus tinctorius* | *Plantago asiatica* | *Lonicera japonica* |
|---|---|---|---|---|
| 1 | 60 | 20 | 10 | 10 |
| 2 | 50 | 20 | 15 | 15 |
| 3 | 50 | 10 | 25 | 15 |
| 4 | 45 | 20 | 30 | 5 |
| 5 | 42 | 25 | 8 | 25 |
| 6 | 40 | 30 | 20 | 10 |
| 7 | 25 | 10 | 40 | 25 |
| 8 | 25 | 15 | 38 | 22 |
| 9 | 25 | 25 | 25 | 25 |
| 10 | 25 | 25 | 5 | 45 |
| 11 | 20 | 40 | 20 | 20 |
| 12 | 20 | 10 | 60 | 10 |
| 13 | 25 | 15 | 38 | 22 |
| 14 | 25 | 25 | 25 | 25 |
| 15 | 25 | 25 | 5 | 45 |
| 16 | 20 | 40 | 20 | 20 |
| 17 | 20 | 10 | 50 | 10 |

### Preparation Example 3.

Crude powder of crude drugs of *Cucurbita moscha ta* seeds, *Carthamus tinctorius, Plantago asiatica,* and *Lonicera japonica* was mixed, the resultant was subjected to extraction with 10 fold amount of water at a temperature of 95 ± 5 °C for 30 minutes. After filtration, the extract was concentrated. To the resultant were added excipients such as reducing maltose, lactose, starch, and the like, and scent and the resultant was subjected to a granulating step to produce fine granules {InterPunch (a registered trademark: manufactured by Sanwell Co., Ltd.)} The composition thereof is shown below.

**Table 2**

| Indication of Nutrient composition of InterPunch per two packages (1.5 g ×2) | |
|---|---|
| Calorie | 11.5 Kcal |
| Protein | 0.042 g |
| Fat | 0.003 g |
| Saccharide | 2.823 g |
| Food fiber | 0.03 g |
| Sodium | 0.444 mg |
| Lactulose | 400 mg |
| *Cucurbita moschata* seeds | Mixed extract |
| *Plantago asiatica* seeds | from 1000 mg in |
| *Carthamus tinctorius* | terms of crude |
| Flower *of Lonicera japonica* | powder |
| Bifidobacterium sp. | 40 mg |

Test Example 1. Inhibiting Production of Total IgE Antibodies in the Blood of Mice Sensitized with Cedar Pollen

The Test was carried out according to the representative method for testing allergy described in Allergy, 42(1), 74-80(1993) with the necessary modifications.

BALB/cCrSlc mice (female) of 12-15 weeks age were used. Inhibiting action on the production of total IgE antibodies in the blood in response to cedar pollen ( (Cedar Pollen Extract-Cj ) (LSL Co., Ltd.)) when the hot water extract of Formulation A was orally administered or intraperitoneally injected, was investigated. The extract for the oral administration group was prepared by adding a 10-fold amount of water to the original crude drugs of Formulation A. Extraction was carried out at 95 ± 5°C for 30 minutes. The extract was continuously centrifuged at 900 × g; the supernatant liquid thereof was further centrifuged at 10,000 × g and the supernatant liquids were combined to produce the extract. The extract for the intraperitoneal injection group was prepared by adding a 10-fold amount of water to the original crude drugs of Formulation A. Extraction was carried out at 95 ± 5°C for 30 minutes. The extract was continuously centrifuged at 900 × g; the supernatant liquid thereof was further centrifuged at 10,000 × g and the supernatant liquids were combined. These supernatant liquids were dialyzed to collect a fraction having a molecular weight of 14,000 or more, then strained through a 0.45 MY Filter and sterilized, resulting in an extract. The test groups were as follows:
Group 1: a non-treated control (negative control group)
Group 2: a group to which only pollen was given intraperitoneally
Group 3: a group to which pollen and extract (0.3 ml) were intraperitoneally administered (single application at the time of 13 days after the initial immunization)
Group 4: a group to which pollen and extract (0.6 ml) were administered per os (consecutively applied per os from 7 days to 12 days after the initial immunization, and 5 mg of cedar pollen and 4 mg of aluminum hydroxide intraperitoneally given)

The method for the measurement of the total number of IgE antibodies was in such a manner that all the blood was collected under anesthetization and the sample serum was separated and preserved at -80°C until the antibody value was measured. A sandwich ELISA method carried out measurement of the total number of IgE antibodies in the conventional manner.

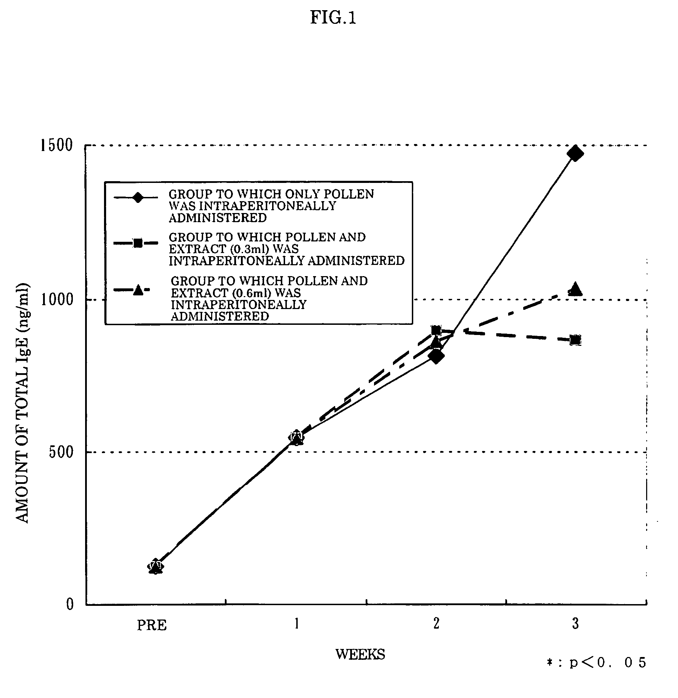

Results of the measurement of the total number of IgE antibodies are shown in Fig. 1. Before administration and in the first and second weeks, there was no significant difference among the groups. However, in the third week, there was a significant increase to 1474.2 ng/ml in the group to which only pollen was given (group 2), while, in the group where pollen and intraperitoneal injection were applied (group 3) (866.97 ng/ml) and in the group to which pollen and oral administration were applied (group 4) (1036.24 ng/ml), the increase was significantly inhibited as compared with the group where only pollen was administered (group 2) (p < 0.05 in a student's t-test). In a comparison between the group where pollen and the Formulation A were intraperitoneally applied (group 3) and the group where pollen and the Formulation A were applied per os (group 4), there was no statistical significance.

From the above results, it was confirmed that the health food containing the present invention inhibited the production of IgE antibodies in the blood of mice sensitized with cedar pollen and was able to inhibit the allergic reaction.

Test Example 2. Preventive or Curative Effects on Patients Suffering From Cedar Pollen Allergy

In order to investigate the efficacy and the safety of health food containing the present invention with regard to pollen allergy, a clinical test was carried out with male and female adult patients suffering from pollen allergy, using the hot water extracts of Formulations A and B in Preparation Example 1. Formulation A was applied to 20 patients suffering from pollen allergy (10 males and 10 females) while Formulation B was given to 72 patients suffering from pollen allergy. The test was started early in February and administration was begun individually by each patient arbitrarily and continued for 90 days. The patients were asked to fill in questionnaires before the start and at the time of termination, and evaluation was carried out in accordance with the answers given. During the test, the hot water extract containing essence equivalent to that found in 1.0 g of the original crude drugs was administered during the test period, administration being recorded every day. Any volunteer who stopped intake during the administration period or any volunteer whose intake was irregular was excluded from the final evaluation of the effects.

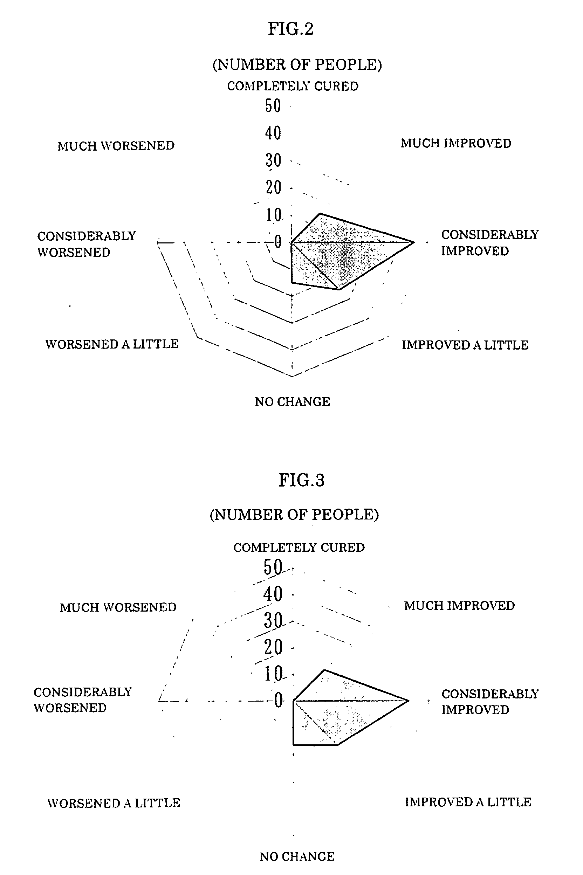

After the test, the patients were asked to report their degree of improvement as compared with their usual symptoms during that season in terms of eight stages: completely cured, much improved, considerably improved, improved a little, no change, worsened a little, considerably worsened and much worsened. The result was that, in both Formulations A and B, one-half or more of the volunteers were "considerably improved" or better, and more than 80% were "improved a little" or better (Figs. 2 & 3).

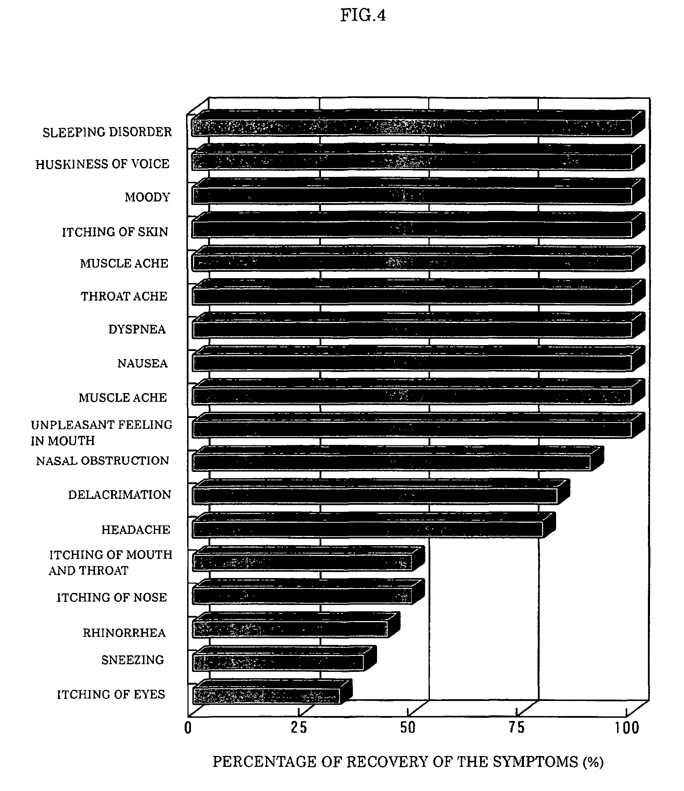

When the usual symptoms of every preceding year until now, and those of this year were compared for the patients to whom the hot water extract of Formulation A was administered, an improvement in the symptoms in nasal obstruction, rhinorrhea, sneezing, itching of the eyes, delacrimation, itching of the nose, huskiness of the voice, sleeping disorders, general reduction in symptoms from previous years, headaches, itching of the mouth and throat, rash, moody, nausea, dyspnea, throat ache, muscle ache and unpleasant feeling in the mouth, was noted (Fig. 4).

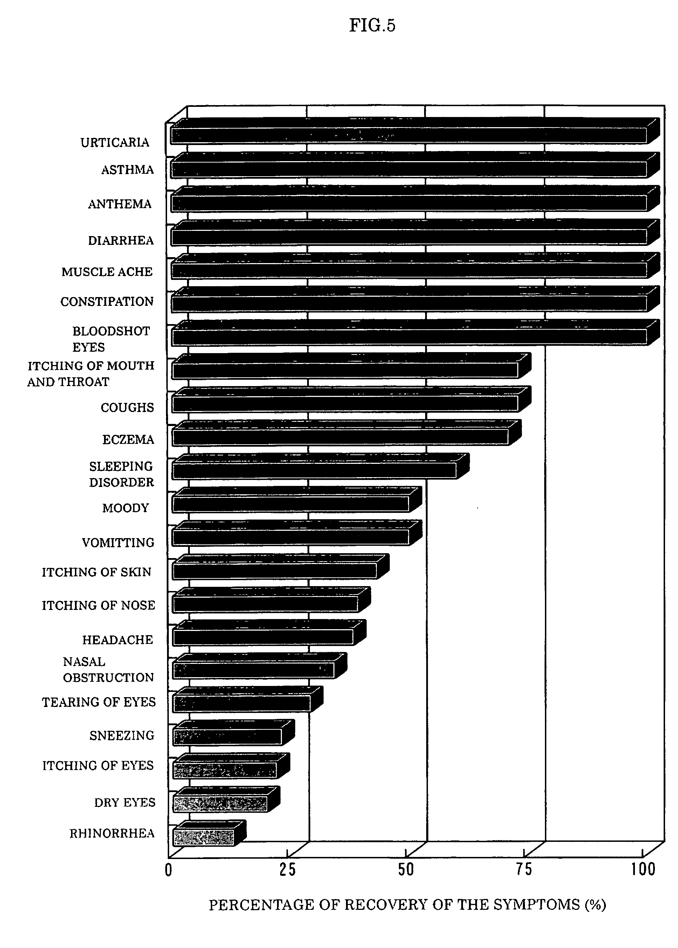

When the usual symptoms experienced in previous years and those of this year were compared for the patients to whom the hot water extract of Formulation B was administered, improvement was noted in 100% of the patients for the symptoms of urticaria, asthma, anthema, diarrhea, muscular ache, constipation and bloodshot eyes, and an improvement in many was noted for (in order of the number improved) itching of the mouth and throat, coughs, eczema, sleeping disorders, moody, vomiting, itching of the skin, itching of the nose, headaches, nasal obstruction, tearing of the eyes, sneezing, itching of the eyes, dry eyes and rhinorrhea (Fig. 5).

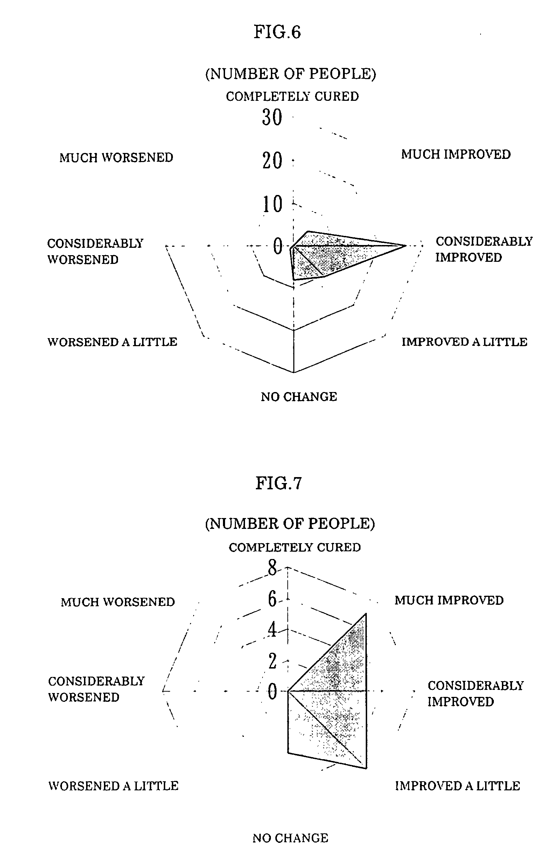

The analysis of the correlation of improvement in symptoms and the starting time of treatment with the hot water extract using Formulation B, showed that when administration was started from a stage before the onset of symptoms (early February), more cases were "considerably improved" or better, than where administration was started after the onset of symptoms (Figs. 6 & 7).

Accordingly, it was confirmed that the combined crude drugs of the present invention had a preventive curative effect.

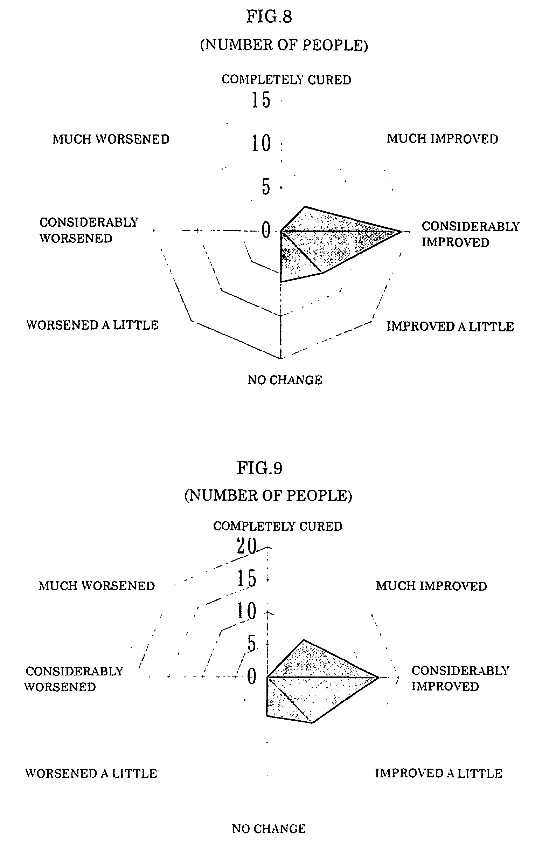

In the degree of improvement of the patients to whom the hot water extract of Formulation B was administered, no influence due to the sexuality of the volunteer was noted (Figs. 8 & 9).

When a comparison was made between cases of combination use with curing by the doctors and other medicament or other health foods, and instances where only the health food in which the Formulation under discussion was used throughout, the degree of improvement was nearly the same. Even when used in combination with other curative approaches and the Formulation, there were no bad effects. In addition, no influence on the degree of improvement by differences in physical constitution was noted.

From the above-mentioned results, it was confirmed that the health food according to the present invention was effective in improving the symptoms of patients suffering from pollen allergies and that, especially when it was taken in a preventive manner before the beginning of the pollen allergies, the onset of symptoms could be inhibited.

Test Example 3. Preventive Effects on Patients Suffering from Pollen Allergies

Health food using Formulation A as in Preparation Example 1 (corresponding to 1 g of the crude powder per day) was given during 2 months beginning in early March to a female patient of 66 years age suffering from pollen allergy and an improvement in the symptom was observed. She was usually troubled by sniffles and itching of the eyes every year. She was given the medicaments ambroxol hydrochloride and progesterone together with the health food, and no symptoms of pollen allergy were noted during that period.

Test Example 4. Curative Effects on Patients Suffering from Nasal Allergy

In order to investigate the efficacy and safety of the health food using the present invention with regard to nasal allergy, a single blind comparative test was carried out on patients suffering from a year-round nasal allergy using the health food of Formulation B in Preparation Example 1 and a placebo having the same appearance and taste as the health food above. In the test, 53 patients suffering from year-round house dust (HD) nasal allergy (18 years age or older were tested, with a proviso that no other medicament be jointly used). The diagnosis was carried out in such a manner that, in accordance with the guidelines for diagnosis of nasal allergy (Guidelines for Treatment of Nasal Allergy - Year-round Rhinitis - Revised Third Edition (1999) - by the Nasal Allergy Diagnosis Guideline Preparation Committee), those who were suffering from sneezing, sniffling and stuffiness, and being positive for two or more of the following: snivel acidophil test, HD intracutaneous reaction, blood HD PAST value and HD nasal induction test, were defined as patients suffering from a throughout-the-year nasal allergy. Patients having the risk of being affected by pollen allergies during the administration period were excluded. In terms of classification according to the degree of severity of the symptoms, there were 25 light patients, 28 medium patients and 8 severe patients. After a control observation period of one week, either the actual treatment or a placebo was administered twice daily for 4 weeks (for each administration, a dosage corresponding to 1.0 g of original crude drugs was used) and the progress of the symptoms was judged from the allergy diaries of the patients. Before and after the administration, a peripheral blood test, hepatic and biliary system, renal function and urine tests were carried out to evaluate whether or not there were side effects. With regard to 18 patients to whom the treatment was administered in December, peripheral blood was collected before and after the administration, and erythrocytes were removed therefrom. Stimulation was applied with a tick antigen (10 ng/ml) to 24 plates, IL-5 and IFNγ in the supernatant liquid of the incubation solution after 7 days were measured by ELISA and, from a comparison with IL-5 and IFNγ in non-stimulated supernatant liquid after incubation, the amount of production of HD-stimulated IL-5 and IFNγ was determined.

After the initiation of the administration of the health food, there were no volunteers who stopped intake and investigation was possible for the 30 patients using the actual treatment and 23 patients using a placebo. Between the actual treatment group and the placebo group, there was no difference in the classification of the severity. Judging the degree of improvement from the patients' allergy diaries, there was an onset of sneezing in 20.0% of the actual treatment group and 30.4% of the placebo group; onset of sniffling was 16.6% in the actual treatment group and 39.1% in the placebo group; onset of stuffiness was 20.0% in the actual treatment group and 30.4% in the placebo group, and daily activities became more difficult in 6.6% of the actual treatment group and 13.0% of the placebo group, so that in all cases the actual treatment group was superior. Expression of significant side effects such as gastrointestinal symptoms was not noted in either actual treatment or placebo groups. Further, there was no abnormal data in the general blood tests.

Investigation of production of cytokine using peripheral blood was carried out on 11 patients for the actual treatment group and 7 patients for the placebo group with regard to IFN-γ while, with regard to IL-5; it was carried out on 6 patients in the actual treatment group and 6 patients in the placebo group. In the actual treatment group, production of IFN-γ caused by HD stimulation significantly increased after administration of the formulation, as compared with IFN-γ production before administration (p < 0.05), while, in the placebo group, no significant change was noted. Production of IL-5 caused by HD stimulation significantly decreased (p < 0.05) in the group using the formulation while, in the placebo group, only a slight but not significant decrease was noted. Accordingly, it was strongly suggested that health food using the present invention had a Th1 cytokine promoting action and a Th2 cytokine suppressing action.

From the above result, it was confirmed that health food according to the present invention was effective for the improvement of symptoms of patients suffering from HD induced nasal allergy, and was safe as well. Its main action is believed to be an adjustment and improvement in an abnormal balance between Th1 and Th2 cytokines, which is a cause of immediate type hypersensitivity.

Test Example 5. Curative Effects on Patients Suffering from Year-round Nasal Allergy

Health food according to Formulation A in Preparation Example 1 (corresponding to 1 g of the original crude drug per day) was administered to a male patient 4 years of age suffering from a year-round nasal allergy, and improvement in the symptoms was observed. In the initial stage, severe stuffiness was noted, but after the administration, much nasal mucus was discharged and the stuffiness was reduced. When the administration was stopped, his nose became stuffy again after one week.

### Test Example 6. Curative Effects on Atopic Dermatitis

Health food using Formulation A in Preparation Example 1 (corresponding to 1 g of the original crude drug per day) was administered to a female patient of 27 years' age suffering from atopic dermatitis and improvement in the dermal symptoms was observed. Until that time, the patient had undergone curative that included medicament, the intake of food devoid of allergens, and other health foods, but no apparent improvement in the symptoms was noted. When an observation was made after about one month from the beginning of administration of health food using the formulation, flaring of the skin, exudations, itching, etc., were reduced.

From the above results, it was confirmed that the health food of the present invention was effective in producing reductions in the symptoms of Atopic dermatitis.

### Test Example 7. Curative Effects on Infantile Asthma

Health food according to Formulation A in Preparation Example 1 (corresponding to 1.5 g of the original powder per day) was administered to a male patient of 7 years age suffering from infantile asthma and improvement in the symptoms was observed. Until that time, curative had been done by medicament, but when he was exposed to dust; symptoms of asthma were immediately noted. After the administration of health food containing the formulation however, his cough decreased and stuffiness was considerably diminished.

### Test Example 8. Curative Effects on Urticaria

After an abortion a half-year previously, a female patient of 23 years age complained of map-like urticaria on the soft areas of the skin, mostly on the abdomen, hands and feet, every night. However, when health food according to Formulation A in Preparation Example 1 (three times a day; each corresponding to 1.0 g of crude powder) was administered and the symptoms were observed for six months, little by little a reduction in the symptoms during the period of administration was noted.

### Test Example 9. Test for Confirmation of Safety

Safety of the Formulations A and B in Preparation Example 1 was investigated in humans.

Health food according to the present invention was administered twice daily (corresponding to 1.0 g of crude drugs per administration) to 7 healthy and normal male adults. Before starting the administration, and after one and then two weeks from the start, blood was collected and general clinical tests (i. e. hematological tests; white blood cell count, Red blood cell count, hemoglobin, hematocrit, MCV, MCH, MCHC, platelet count and differential white blood count), biochemical tests of blood (total protein, albumin, A/G, total bilirubin, MCV, MCH, MCHC, GOT, GPT, alkaline phosphatase, γ-GTP, total cholesterol, neutral fat, urea nitrogen, uric acid and creatinine), immunobiochemical tests (nonspecific IgE, nonspecific IgG and transferrin) and auscultation, percussion and physical tests (body temperature, pulse and blood pressure) were carried out by medical doctors whereby the safety of the health food of the present invention was investigated. The volunteers were also interviewed.

The result was that, during the two-week period of test administration, no harmful cases seemingly caused by administration of the tested food were noted in subjective symptoms, objective findings and clinical test data, including immunobiochemical tests. It was confirmed that the health foods in Preparation Example 1 were of no problem in both Formulations A and B.

### Test Example 10. Safety as Health Food

Health food of the present invention was given to a healthy and normal male adult for six years. At the initiation of the administration, he was 31 years of age. During the first two years, a compound of crude drug powder of Formulation A was given at a dose of 1 g on average per day and, after that, health food containing Formulation A was given at a dose equivalent to 1 g of the original powder on average per day. The result was that, during the period of administration, no negative health effects were noted in general blood properties and state of health.

### Test Example 11. Safety when used during Pregnancy

The Formulation B of Preparation Example 1 according to the present invention was administered to a female of 29 years' age during pregnancy and no ill effects on her state of health were noted during pregnancy, and upon and after delivery. The newborn male baby was also normal and has been growing in a healthy state subsequently.

Test Example 12. Curative Effects on Dermatitis in Animals

The tablets (containing 1.0 g of crude powder per 4 tablets) or fine granules (containing 1.0 g of crude powder per 1.0 g of fine granules) prepared from the Formulation A of Preparation Example 1 according to the present invention were administered for a period of one month to 22 dogs (age: from half year after born to 14 years old; sexuality: five male dogs and seventeen female dogs; breed: five Pugs, three Shiba Inu dogs, three Malteses, three mixed breed dogs, two Shih Tzus, one Cavalie King Charles Spaniel, one Boston Terrier, one Japanese dog, one Miniature dachshund, one French bulldog, and one Labrador Retriever) suffering from dermatitis which had high IgE antibody levels and visited regularly a veterinary doctor so as to examine the reducing effects of the present inventive crude drugs against the dermatitis. As daily doses, were administered, as a standard dose, two tablets for dogs having body weight of less than 5 kg, four tablets for ones having body weight of 5 to 10 kg, or eight tablets for ones having body weight of 10 to 20 kg; or 0.5 g, 1.0g or 2.0g, respectively in the case of fine granules. The items for the evaluation are 1. Erythema, 2. Dry skin and desquamation, or the like, 3. Papula, and 4. Pruritus, and these items were evaluated by five ranks from 1 to 5, respectively. The veterinary doctor judged the whole states of the dermatitis, with 6 categories; 1. Disappearing of the symptoms, 2. Remarkably improved, 3. Improved, 4. Slightly improved, 5. No change, and 6. Worsened from the above-mentioned evaluation results. Among them, 12 dogs were administered singly with the present inventive crude drug without any commercial medicine for the observation of the states of dermatitis, and there was observed no dog whose dermatitis became worse. Two dogs were remarkably improved, 2 dogs were improved, 6 dogs were slightly improved, and 2 dogs showed no change. Ten dogs were subjected to the curative treatment of the present inventive crud drugs with concomitant use with commercial medicines such as steroid type drugs. 2 dogs were remarkably improved, one dog was improved, three dogs were slightly improved, and 4 dogs showed no change among them. There was no case whose dermatitis was worsened. The improved cases were observed in all of the symptoms of items evaluated. Among the cases wherein the commercial medicines were administered, there were confirmed such effects that the dosages of the medicines were reduced to a great extent. It has been recognized that the present inventive composition shows also good reducing effects of the dermatitis due to the allergy in dogs, from the above-mentioned results.

### Industrial Applicability

In accordance with the present invention, there is provided a safe and excellent curative agent for pollen allergy, allergic rhinitis, atopic dermatitis, asthma or urticaria of humans and companion animals. Especially for pollen allergy, it can be used as a preventive agent which is able to inhibit the onset of symptoms when administered before the beginning of it and thereafter. In addition, there is no risk of side effects and it can be commonly used for a long period, not only as medicament, but also as a functional food, dietary supplement or health food.

## Claims

1. A preventive or curative composition for pollen allergy, allergic rhinitis, atopic dermatitis, asthma or urticaria; containing two kinds of crude drugs of *Cucurbita moschata* seeds and *Carthamus tinctorius,* and at least one crude drug selected from *Plantago asiatica, Lonicera japonica, Glycyrrhiza uralensis, Coix lachrymal-jobi* var. *ma-yuen, Zingiber officinale, Curcuma longa, Curcuma zedoaria* and *Artemisia argyi.*

2. A composition as recited in claim 1, which is used for preventive or curative treatment of allergic rhinitis, asthma or urticaria.

3. A preventive or curative composition as recited in claim 1 or 2, which contains four kinds of crude drugs of *Cucurbita moschata* seeds, *Carthamus tinctorius, Plantago asiatica,* and *Lonicera japonica* as an effective component.

4. A preventive or curative composition for pollen allergy, which is used for being administered to a mammal who suffers from pollen allergy every year before the season of pollen allergy; containing two kinds of crude drugs of *Cucurbita moschata* seeds, *Carthamus tinctorius,* and at least one crude drug selected from *Plantago asiatica, Lonicera japonica, Glycyrrhiza uralensis, Coix lachrymal-jobi* var. *ma-yuen, Zingiber officinale, Curcuma longa, Curcuma zedoaria* and *Artemisia argyi* as an effective component.

5. A preventive composition as recited in claim 4, which contains four crude drugs of *Cucurbita moschata* seeds, *Carthamus tinctorius, Plantago asiatica, Lonicera japonica* as an effective component.

6. A preventive composition as recited in any one of claim 4 or 5, wherein the mammal is human being.

7. A method for preparing a preventive or curative composition for pollen allergy, allergic rhinitis, atopic dermatitis, asthma or urticaria; containing two kinds of crude drugs of *Cucurbita moschata* seeds and *Carthamus tinctorius,* and at least one crude drug selected from *Plantago asiatica, Lonicera japonica, Glycyrrhiza uralensis, Coix lachrymal-jobi* var. *ma-yuen, Zingiber officinale, Curcuma longa, Curcuma zedoaria* and *Artemisia argyi.*

8. A method for preparing a preventive or curative composition for pollen allergy which is used for administering two kinds of crude drugs of *Cucurbi ta moschata* seeds, *Carthamus tinctorius,* and at least one crude drug selected from *Plantago asiatica, Lonicera japonica, Glycyrrhiza uralensis, Coix lachrymal-jobi* var. *ma-yuen, Zingiber officinale, Curcuma longa, Curcuma zedoaria* and *Artemisia argyi* to a patient who suffers from pollen allergy every year before the season of pollen allergy.

9. A health food or a functional food for the prevention of or the curative for pollen allergy, allergic rhinitis, atopic dermatitis, asthma or urticaria by administering two kinds of crude drugs of *Cucurbita moschata* seeds and *Carthamus tinctorius,* and at least one crude drug selected from *Plantago asiatica, Lonicera japonica, Glycyrrhiza uralensis, Coix lachrymal-jobi* var. *ma-yuen, Zingiber officinale, Curcuma longa, Curcuma zedoaria* and *Artemisia argyi* and *Lonicera japonica* as an effective component.

10. A health food or a functional food as recited in claim 9 which contains four kinds of crude drugs of *Cucurbita moschata* seeds, *Carthamus tinctorius, Plantago asiatica,* and *Lonicera japonica*.

11. A composition as recited in any one of claims 9 to 10 wherein the composition is used for preventing from, or improving or lightening pollen allergy, allergic rhinitis, atopic dermatitis, asthma or urticaria.

12. A health food or a functional food for the prevention of pollen allergy which contains two kinds of crude drugs of *Cucurbi ta moschata* seeds and *Carthamus tinctorius,* and at least one crude drug selected from *Plantago asiatica, Lonicera japonica, Glycyrrhiza uralensis, Coix lachrymal-jobi* var. *ma-yuen, Zingiber officinale, Curcuma longa, Curcuma zedoaria* and *Artemisia argyi* and *Lonicera japonica* as an effective component.

13. A health food or a functional food as recited in claim 12 which contains four kinds of crude drugs of *Cucurbita moschata* seeds , *Carthamus tinctorius, Plantago asiatica,* and *Lonicera japonica.*

14. An animal health food or an animal functional food for the prevention of pollen allergy allergic rhinitis, atopic dermatitis, asthma or urticaria, which contains two kinds of crude drugs of *Cucurbita moschata* seeds and *Carthamus tinctorius,* and at least one crude drug selected from *Plantago asiatica, Lonicera japonica, Glycyrrhiza uralensis, Coix lachrymal-jobi* var. *ma-yuen, Zingiber officinale, Curcuma longa, Curcuma zedoaria* and *Artemisia argyi* and *Lonicera japonica* as an effective component.

15. A composition, a health food, or a functional food as recited in any one of claims 1 to 14, wherein two crude drugs of 20 to 60 % by weight of *Cucurbita moschata* seeds and 10 to 40 % by weight of *Carthamus tinctorius,* and 5 to 50 % by weight of at least one crude drug selected from *Plantago asiatica, Lonicera japonica, Glycyrrhiza uralensis, Coix lachrymal-jobi* var. *ma-yuen, Zingiber officinale, Curcuma longa, Curcuma zedoaria* and *Artemisia argyi* is a compounding ratio therein.

16. A composition, a health food, or a functional food as recited in claim 15, wherein 30 to 50 % by weight of the *Cucurbita moschata* seeds, 10 to 30 % by weight of *Carthamus tinctorius,* 5 to 30 % by weight of *Plantago asiatica,* and 5 to 30 % by weight of *Lonicera japonica* is a compounding ratio therein.
